(19) Europäisches Patentamt
European Patent Office
Office européen des brevets

(11) **EP 2 422 351 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**18.05.2022   Patentblatt 2022/20**

(45) Hinweis auf die Patenterteilung:
**22.06.2016   Patentblatt 2016/25**

(21) Anmeldenummer: **10716482.4**

(22) Anmeldetag: **20.04.2010**

(51) Internationale Patentklassifikation (IPC):
**H01J 37/244** (2006.01)      **H01J 37/304** (2006.01)
**H01J 33/02** (2006.01)      **B65B 55/08** (2006.01)
**A61L 2/08** (2006.01)

(52) Gemeinsame Patentklassifikation (CPC):
**H01J 37/244; H01J 37/304;** A61L 2/087;
H01J 2237/164; H01J 2237/2441; H01J 2237/24507

(86) Internationale Anmeldenummer:
**PCT/EP2010/002396**

(87) Internationale Veröffentlichungsnummer:
**WO 2010/121775 (28.10.2010 Gazette 2010/43)**

(54) **VERFAHREN UND VORRICHTUNG ZUR ÜBERWACHUNG DER INTENSITÄT EINES ELEKTRONENSTRAHLS**

METHOD AND DEVICE FOR MONITORING THE INTENSITY OF AN ELECTRON BEAM

PROCÉDÉ ET DISPOSITIF DE CONTRÔLE DE L'INTENSITÉ D'UN FAISCEAU ÉLECTRONIQUE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **21.04.2009   DE 102009018210**

(43) Veröffentlichungstag der Anmeldung:
**29.02.2012   Patentblatt 2012/09**

(73) Patentinhaber: **KHS GmbH**
**44143 Dortmund (DE)**

(72) Erfinder:
• **KEIL, Gernot**
**55595 Braunweiler (DE)**
• **MONZEL, Alois**
**67591 Mörstadt (DE)**

(56) Entgegenhaltungen:
EP-A1- 0 583 974      EP-A1- 1 376 155
WO-A1-01/14911      DE-A1- 4 108 768
DE-A1-102008 045 187      DE-A1-102009 018 210
DE-B- 1 122 739      DE-B2- 2 920 901
DE-C2- 3 590 146      DE-T2- 60 017 689
DE-T2- 69 621 382      JP-A- 2005 024 307
US-A- 2 717 964      US-A- 3 729 632
US-A- 4 652 763      US-A1- 2004 113 084
US-A1- 2004 227 095      US-A1- 2008 073 549
US-B1- 6 221 216      US-B1- 6 429 444
US-B2- 7 375 345

• KNEELAND D R ET AL: "Industrial use of the real time monitor for quality assurance in electron processing<1>" RADIATION PHYSICS AND CHEMISTRY, ELSEVIER SCIENCE PUBLISHERS BV., AMSTERDAM, NL LNKD-DOI:10.1016/S0969-806X(99)00190-5, Bd. 55, Nr. 4, 11. Juli 1999 (1999-07-11), Seiten 429-436, XP004169518 ISSN: 0969-806X

• BOHNE W ET AL: "Beam-current measurement based on residual gas ionization" NUCLEAR INSTRUMENTS & METHODS IN PHYSICS RESEARCH, SECTION - B:BEAM INTERACTIONS WITH MATERIALS AND ATOMS, ELSEVIER, AMSTERDAM, NL LNKD-DOI:10.1016/0168-583X(95)01311-3, Bd. 113, Nr. 1, 1. Juni 1996 (1996-06-01), Seiten 78-80, XP004007769 ISSN: 0168-583X

• GLENN F. KNOLL: "Radiation Detection and Measurement", 1999 pages xi-xiv,

• NABLO S. V. et al.: "Real Time Monitoring of Electron Processors", Radiation Physics and Chemistry;, vol. 46, 12 September 1995 (1995-09-12), pages 1377-1383,

• KNEELAND D. R. et al.: "Industrial Use of the Real Time Monitor for Quality Assurance in Electron Processing", Radiation Physics and Chemistry;, vol. 55, 11 July 1999 (1999-07-11), pages 429-436,

• ICKE et al.: "Advanced Electron Beams", Cold, Dry Sterilization of PET Bottles for Aseptic Applications, PET Strategies Conference 2007, 30 November 2007 (2007-11-30),

- **"PET Strategies Conference Program Agenda", Packaging Strategies Conferences, 28 November 2007 (2007-11-28), Ponte Vedra Beach Retrieved from the Internet: URL:http://web.archive.org/web/20071008220 720/www.bnpevents.com/PS/2007/PET/Program A genda.html**
- **"Information and Registration Brochure", PET Strategies Conference 2007, 28 November 2007 (2007-11-28), Vedra Beach Beach**
- **Conference Workbook PET Strategies 2007**

- **"PET Strategies Conference Program Agenda", Packaging Strategies Conferences, 28 November 2007 (2007-11-28), Ponte Vedra Beach Retrieved from the Internet:**

## Beschreibung

Die Erfindung betrifft ein Verfahren zur Überwachung der Intensität eines Elektronenstrahles.

Die Erfindung betrifft darüber hinaus eine Vorrichtung zur Überwachung der Intensität eines Elektronenstrahles.

Derartige Elektronenstahlen können beispielsweise in röhrenförmigen Vorrichtungen erzeugt werden, die ähnlich zu Röntgenröhren ausgebildet sind und innerhalb derer ein glühendes metallisches Emissionselement angeordnet ist. Die Elektronen werden hierbei durch Glühemission erzeugt und innerhalb der Röhre auf hohe kinetische Energien beschleunigt. Die entsprechend beschleunigten Elektronen treten am Ende der Beschleunigungsstrecke durch ein Austrittsfenster aus der röhrenförmigen Einrichtung aus. Typischerweise wird das Austrittsfenster dünn bzw. schmal ausgebildet und elektrisch auf Erdpotential gelegt. Derartige Einrichtungen werden auch als Elektronenkanonen bezeichnet.

Nach dem Verlassen des Austrittsfensters gelangen die Elektronen in die atmosphärische Umgebung und pflanzen sich dort fort. Eine maximale Ausbreitungsentfernung wird durch die kinetische Energie der Elektronen vorgegeben.

Gemäß einer typischen Ausführungsform wird ein Elektronenstrom generiert, der eine Intensität im Bereich von 100 **pLA** (Mikroampere) bis 200 mA (Milliampere) aufweist. Es wird ebenfalls typischerweise ein kontinuierlicher Betrieb der Röhren vorgesehen, um eine konstante Emissionsrate an Elektronen zu erreichen. Ebenfalls wird typischerweise eine konstante Beschleunigungsspannung verwendet, um einen zeitlich invariablen Elektronenstrom zu generieren.

Aufgrund der Konstruktion der verwendeten Röhren treten zeitlich jedoch kleinere Spannungseinbrüche auf, die beispielsweise durch Sparks entlang der Oberfläche von keramischen Isolatoren verursacht sein können. Derartige Spannungseinbrüche wirken elektrisch wie ein kurzzeitiger Kurzschluss und führen dazu, dass die kinetische Energie der Elektronen des Elektronenstromes abnimmt, womit auch die Strahlintensität und/oder die Reichweite der Elektronen abnehmen können.

Es ist ebenfalls nicht sicher auszuschließen, dass durch derartige Einflüsse ein kurzzeitiger, vollständiger Ausfall des Elektronenstrahles auftritt. In Abhängigkeit von den jeweiligen Ursachen kann ein derartiger Ausfall in einem Zeitbereich von wenigen Mikrosekunden bis zu einigen Millisekunden liegen. Ausfallzeiten, die länger als etwa 1 Millisekunde dauern, können durch eine Überwachung des die Röhre versorgenden Netzgerätes hinsichtlich des Spannungs- und Stromverlaufes erkannt werden. Kürzere Ausfallzeiten werden jedoch aufgrund von Zeitkonstanten in der Filterung des Netzteiles und aufgrund realisierter Abtastraten der Überwachung des Spannungs- und Stromverlaufes nicht oder zumindest nicht zuverlässig erkannt.

Eine Anwendung derartiger Elektronenstrahlen kann beispielsweise zur Entkeimung von Oberflächen eines Packstoffes erfolgen. Bei einer derartigen Anwendung wird die Oberfläche des Packstoffes durch eine geeignete Ablenkung des Elektronenstrahles und/oder durch eine Bewegung des Packstoffes relativ zur Elektronenquelle zumindest in vorgegebenen Bereichen vollständig vom Elektronenstrahl bestrichen, um eine zuverlässige Entkeimung durchzuführen. Bel Intensitätseinbrüchen des Elektronenstrahles, bei einem kurzzeitigen Ausfall des Elektronenstrahles oder bei einem Energieeinbruch des Elektronenstrahls kommt es somit ohne entsprechende Gegenmaßnahmen zu einer unvollständigen Durchführung des Entkeimungsvorganges, was nicht hingenommen werden kann.

In technischer Hinsicht erweist es sich als äußerst schwierig, die Intensitätseinbrüche, Energieeinbrücke bzw. kurzfristige Ausfälle des Elektronenstrahles unter Einhaltung wirtschaftlicher Randbedingungen zuverlässig zu vermeiden. Es wird deshalb angestrebt, entsprechende Intensitätsschwankungen schnell und zuverlässig zu erkennen, um auf die Ablenkung des Elektronenstrahles bzw. den Verlauf der durchgeführten Bewegungen derart einwirken zu können, dass jeder Bereich der zu entkeimenden Oberfläche des Packstoffes ausreichend lange mit einem ausreichend intensiven Elektronenstrahl beaufschlagt wird.

Vorrichtungen und Verfahren zur Kontrolle und Überwachung von Elektronenstrahlen wurden beispielsweise durch den Aufsatz "Industrial use of the real time monitor for quality assurance in electron prosessing" vorgestellt. Dieser Aufsatz schlägt vor, die Behandlung mit Elektronenstrahlen dadurch zu überwachen, dass durch Abbremsen der Elektronenstrahlen erzeugte Röntgenstrahlung mittels eines Röntgensensors erfasst wird.

Mehr im Detail wird vorgeschlagen, die Röntgenstrahlung entweder direkt bei ihrer Entstehung oder aber nach dem Durchdringen einer Strahlungsabschirmung zu erfassen. Ein Beobachten des Auftreffens der Strahlung auf die zu behandelnde Oberfläche bzw. den zu behandelnden Packstoff wird nicht vorgeschlagen.

Im Rahmen der vorliegenden Erfindung ist unter dem Begriff Packstoff jeglicher Stoff zu verstehen, der zur Verpackung oder Aufnahme von verderblichen Gütern geeignet ist. Dabei sind sowohl flexible Stoffe, wie z.B. Folien, als auch starre Stoffe, wie beispielsweise aus Glas bestehende Flaschen oder Gläser, als auch halbstarre Stoffe, wie beispielsweise Kunststoffflaschen, Kunststoffbecher usw. zu verstehen.

Aufgabe der vorliegenden Erfindung ist es daher, ein Verfahren der einleitend genannten Art derart anzugeben, dass eine zuverlässige Intensitätsüberwachung durchgeführt werden kann.

Diese Aufgabe wird erfindungsgemäß durch ein Verfahren nach Anspruch 1 gelöst.

Bei der elektromagnetischen Strahlung, welche lediglich indirekt durch den Elektronenstrahl erzeugt wird, handelt es sich beispielsweise um durch die nachfolgend noch ausführlich beschriebenen Rekombinationen von Elektronen-Ionen-Paaren und den dabei entstehenden elektromagnetischen Strahlungen.

Weitere Aufgabe der vorliegenden Erfindung ist es, eine Vorrichtung der einleitend genannten Art derart zu konstruieren, dass eine schnelle und zuverlässige Intensitätsüberwachung realisiert wird.

Diese Aufgabe wird erfindungsgemäß durch eine Vorrichtung nach Anspruch 11 gelöst.

Die Erfindung wird nachfolgend an einem Ausführungsbeispiel beschrieben. Im Einzelnen zeigen die

Figur 1    in einem Schaubild die dem Fachmann bekannten Verhältnisse des Energieverlustes von Elektronen beim Durchdringen von Luft, und die

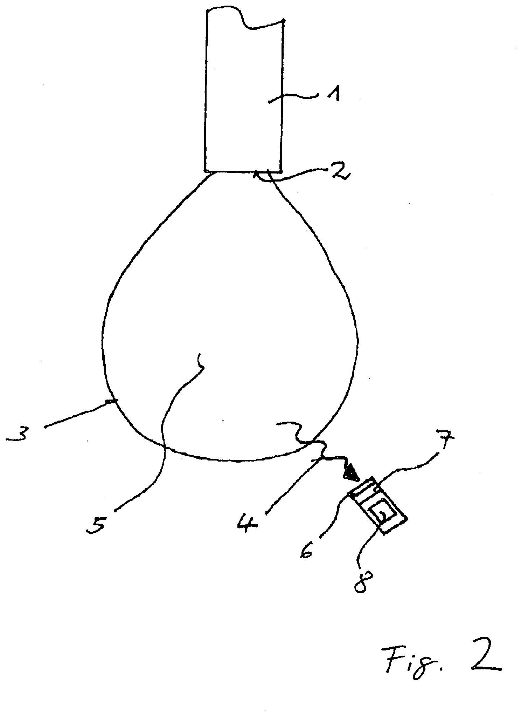

Figur 2    in einer vereinfachten Übersichtsdarstellung eine erfindungsgemäße Vorrichtung.

Eine messtechnische Einrichtung zur Überwachung der vom Elektronenstrahl emittierten elektromagnetischen Strahlung kann mit kleinen räumlichen Dimensionen und preiswert realisiert werden. Ein entsprechender Detektor, der die Strahlung des vom Elektronenstrahl generierten Plasmas detektiert, weist einen sehr niedrigen Fehler auf, so dass bereits Mess- oder Beobachtungszeiten von Bruchteilen von Millisekunden (Zeitkonstanten) zur Signalfilterung, d.h. zum Erkennen einer Veränderung des Elektronenstrahls ausreichen.

Der Detektor kann außerhalb des Elektronenstrahles angeordnet werden, so dass seine thermische Belastung gering ist, was sich überaus positiv auf die Haltbarkeit eines solchen Detektors auswirkt.

Die Intensität der emittierten elektromagnetischen Strahlung korreliert unmittelbar mit der Intensität des Elektronenstrahles und die messtechnische Erfassung der elektromagnetischen Strahlung kann mit einer äußerst guten absoluten Genauigkeit durchgeführt werden.

Eine messtechnische Erfassung in einem charakteristischen Frequenzbereich erfolgt dadurch, dass eine Ultraviolett-Strahlung ausgewertet wird.

Ebenfalls ist daran gedacht, dass eine Lichtstrahlung ausgewertet wird.

Eine besonders einfache konstruktive technische Realisierung wird dadurch unterstützt, dass die elektromagnetische Strahlung während einer Ausbreitung des Elektronenstrahles innerhalb von atmosphärischer Luft ausgewertet wird.

Gemäß einer bevorzugten Anwendung ist daran gedacht, dass der Elektronenstrahl zur Keimreduktion im Bereich einer Oberfläche eines Packstoffes verwendet wird.

Insbesondere ist vorgesehen, dass die Keimanzahl im Bereich der Oberfläche eines Behälters reduziert wird.

Eine kompakte Konstruktion wird dadurch unterstützt, dass die elektromagnetische Strahlung von einem Halbleitersensor detektiert wird.

Zu einer preiswerten Realisierung trägt es bei, dass als Halbleitersensor eine lichtempfindliche Diode, die auch Photodiode genannt wird, verwendet wird.

Eine weitere Ausführungsvariante besteht darin, dass als Halbleitersensor ein CCD Chip, ein lichtempfindlicher CMOS-Baustein oder ein Phototransistor verwendet wird. Auch eine Ausführungsvariante, die einen lichtempfindlichen Widerstand verwendet, ist denkbar.

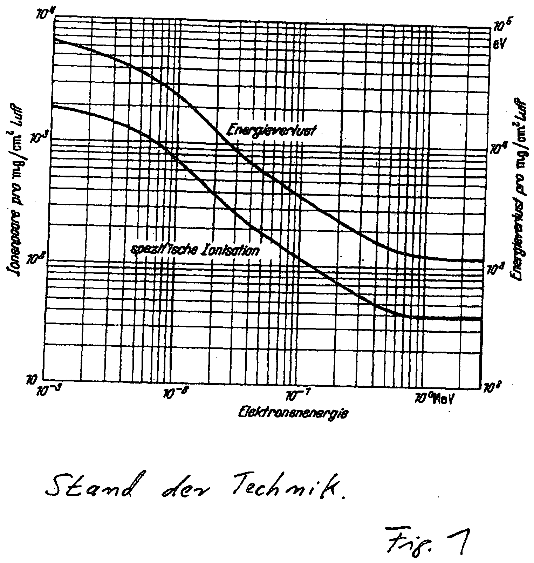

In der Figur 1 sind typische Verläufe einer spezifischen Ionisation und eines Energieverlustes über der Elektronenenergie aufgetragen. Dabei gibt die Kurve, die mit spezifischer Ionisation bezeichnet ist, die Anzahl der durch ein Elektron mit einer Energie von 1 keV bis 3 MeV gebildeten Ionen-Paare bei der Durchstrahlung einer Luftsäule von 1 mg!cm$^2$ wieder. Unter einem einzelnen Ionen-Paar ist hier ein Ensemble aus einem Elektron und einem Ion zu verstehen, was im Folgenden als Elektron-Ionen-Paar bezeichnet wird.

Die Kurve, die mit Energieverlust bezeichnet ist, beschreibt den Energieverlust von Elektronen in Luft bei der Durchstrahlung einer Luftsäule mit einer Flächenmasse von 1 mg/cm$^2$. Üblicherweise werden Effekte, die bei der Wechselwirkung von Elektronen mit Materie auftreten, gerne auf die Flächenmasse bezogen, da unabhängig von der Art der Materie, also unabhängig vom so genannten Absorbermaterial - gleiche Flächenmassen unterschiedlicher Absorber zu annähernd gleichen Effekten führen. So ist bei der Durchstrahlung einer Argonsäule oder einer Stickstoffsäule der gleichen Flächenmasse 6 ein annähernd gleicher Energieverlust AE des Elektronenstrahls zu erwarten, wie bei der Durchstrahlung einer Luftsäule der gleichen Flächenmasse.

Auch die Anzahl der erzeugten Elektron-Ionen-Paare folgt annähernd dieser Gesetzmäßigkeit. Unter "annähernd" ist hier zu verstehen, dass die zu erwartenden Abhängigkeiten materialspezifisch für Absorberzusammensetzungen mit Ordnungszahlen Z zwischen 6 und 20 um lediglich $\pm$ 15% variieren können. Für alle anderen Absorber sind die Abweichungen zwischen den einzelnen Materialien größer. Die Effekte des Energieverlustes von Elektronen in Absorbern werden durch die Bethe-Bloch-Gleichung beschrieben, welche in "Dosimetrie ionisierter Strahlung", Reich B.G. Teubner, Stuttgart 1990, S. 69 erläutert ist.

Eine bevorzugte Anwendung für das erfindungsgemäße Verfahren und die erfindungsgemäße Vorrichtung erfolgt im Zusammenhang mit der Überwachung der Intensität eines Elektronenstrahles, der für die Entkeimung von Packstoffoberflächen verwendet wird. Für derartige Bestrahlungszwecke werden innerhalb einer Röhre zur Erzeugung des Elektronenstrahles die Elektronen derart beschleunigt, dass eine Elektronenenergie im Bereich von 60 keV bis 2 MeV realisiert wird. Die Elektronenstrahlung tritt im Bereich eines Austrittsfensters aus der Röhre aus und gelangt in eine atmosphärische Umgebung oder in eine Umgebung, die aus einem Fremdgas wie beispielsweise Argon oder Stickstoff besteht, und die atmosphärische Umgebung ersetzten könnte, um im Bereich des Packstoffes Sauerstofffreiheit zu erlangen.

Im Bereich des Austrittsfensters wird die Energie der Elektronen reduziert, wobei das Ausmaß dieser Reduktion von der Dicke des Austrittsfensters, dem Material des Austrittsfensters und der kinetischen Energie der Elektronen abhängig ist.

Nach dem Durchlaufen des Austrittsfensters erleiden die Elektronen beim Durchlaufen der atmosphärischen Umgebung einen weiteren permanenten Energieverlust. Dieser Energieverlust entsteht im Wesentlichen durch Impulsübertragung, wobei durch die austretenden Elektronen beispielsweise Elektronen-Ionen-Paare, angeregte Ionen, angeregte Moleküle, Molekülbruchstücke und Radikale erzeugt werden. Insgesamt erzeugen die austretenden Elektronen in der atmosphärischen Umgebung ein Plasma. Generell kann der Bereich, in dem der Elektronenstrahl der Elektronenkanone abgebremst wird, auch von anderen Gasen als Luft, beispielsweise von Argon, Stickstoff, Helium oder dergleichen ausgefüllt sein. Das gilt auch für die nachfolgenden Beschreibungen.

In der Figur 1 ist beispielhaft die Erzeugungsrate von Elektronen-Ionen-Paaren als spezifische Ionisation dargestellt. Ebenfalls ist der Energieverlust von Elektronen in einem Energiebereich zwischen 1 keV und 3 MeV für eine Absorptionsstrecke von 1 mg/cm$^2$ Luft dargestellt, was bei einer Temperatur von 293 Kelvin und einem Druck von 1013,25 mbar einer Luftsäule von etwa 8,3 mm entspricht. Die entsprechenden Energieverluste führen dazu, dass ein Elektronenstrahl von beispielsweise 130 keV in atmosphärischer Luft eine Ausbreitungsstrecke von etwa 20 cm aufweist. Packstoffoberflächen, die relativ zum Austrittsfenster eine Entfernung von mehr als 20 cm aufweisen, werden somit von einem Elektronenstrahl mit dieser Energie nicht mehr erreicht.

Zur Lösung der Aufgabe der vorliegenden Erfindung macht sich die vorliegende Erfindung den Effekt zu nutze, dass zumindest ein wesentlicher Teil der ursprünglichen kinetischen Energie der Elektronen dadurch verbraucht wird, dass die sich vom Austrittsfenster aus fortpflanzenden Elektronen ihre Bewegungsenergie durch Impulse auf die von ihnen durchdrungene Atmosphäre übertragen.

Dabei werden in Fortbewegungsrichtung der Elektronen durch die Stöße mit den Bestandteilen der die Elektronen umgebenden Atmosphäre permanent Elektronen aus den Hüllen der Atome oder Moleküle dieser Bestandteile herausgeschlagen, wodurch wiederum Paare von freien Elektronen und Ionen gebildet werden. Der Elektronenstrahl erzeugt also während seiner Ausbreitung permanent ein Plasma.

Aus der Fachliteratur, beispielsweise H. Kuchling, "Taschenbuch der Physik,,, Carl Hanser Verlag, 16. Auflage, Seite 572 ist bekannt, dass die mittlere Ionisierungskonstante für Luft 33,85 eV beträgt, so dass bei bekanntem Energieverlust eines Elektrons bei der Erzeugung eines Elektronen-Ionen-Paares in einem Absorber die Anzahl der insgesamt durch ein Elektron des Elektronenstrahls entlang seines Weges gebildeten Paare berechnet werden kann.

Im Falle eines das Austrittsfenster verlassenden Elektrons mit einer Energie von 130 keV, erlangt dieses Elektron nach Figur 1 zumindest für die erste Strecke von 8,3 mm im Absorber einen Energieverlust von 3,3 keV. Durch die, diesen Energieverlust erzeugenden Stöße werden demnach 97 Elektronen-Ionen-Paare erzeugt. Für alle weiteren zurückgelegten Strecken im Absorber nimmt die Anzahl der gebildeten Elektronen-Ionen-Paare zu, weil der Energieverlust mit abnehmender Elektronenenergie zunimmt. Daher kann die Verwendung eines Wertes von 100 gebildeten Elektronen-Ionen-Paaren in einer Absorberstrecke von 8,3 mm für die nachfolgende Abschätzung als konservativ gelten.

Dass der Nachweis des Elektronenstrahls, bzw. die Überwachung seiner Intensität mit der erforderlichen Genauigkeit erfolgen kann, zeigt die nachfolgende Beispielrechnung:

Ein Elektron mit der Energie von 130 keV erzeugt:

$$24 \text{ mgen'} \cdot 100 \text{ Elektronen-Ionen-Paare pro } (1 \text{ mg/cm}^2) =$$

$$2400 \text{ Elektronen-Ionen-Paare.}$$

Zur Sterilisation von Packstoffen durch Elektronenstrahlen, welche sich durch Luft fortpflanzen, werden beispielsweise Strahlströme im Bereich von 100 **pLA** bis 200 mA angewendet.

Folgende Beispielrechnung bezieht sich auf einen Strahlstrom von 1 mA, mit welchem bei der Sterilisation von Packstoffen gute bis sehr gute Ergebnisse erzielt werden.

Ein Strom von 1 mA entspricht einer Anzahl von $6,25 \cdot 10^{15}$ Elektronen pro Sekunde. Ein Strahlstrom von 1 mA erzeugt also an Luft mindestens $6,25 \cdot 10^{15} \cdot 2400$ Elektronen-Ionen-Paare pro Sekunde. Das entspricht $1,5 \cdot 10^{19}$ Elektronen-Ionen-Paaren pro Sekunde.

Fast alle dieser Elektronen-Ionen-Paare rekombinieren sofort wieder, wobei ein Großteil der bei der Rekombination freiwerdenden Energie in der Form von Strahlung im Bereich von UV-Licht oder sichtbarem Licht - kurz "Lichtstrahlung" - emittiert wird.

Der genaue Anteil der Quanten der Lichtstrahlung an Quanten anderer Bereiche oder an strahlungslosen Übergängen kann nicht angegeben werden, jedoch liegt der Anteil - wie dem Fachmann allgemein bekannt ist - eher bei 50 % als bei 1 %.

Ein Anteil der Rekombination im Bereich der Lichtstrahlung kann deshalb als äußerst konservative Abschätzung angesehen werden. Von den $1,5 \cdot 10^{19}$ Elektronen-Ionen-Paaren pro Sekunde werden also mindestens $1,5 \cdot 10^{17}$ Photonen im Bereich der Lichtstrahlung emittiert.

Die UV- oder Lichtstrahlung erfolgt, ausgehend vom Austrittsfenster des Elektronenstrahls aus einem ballonförmigen Bereich heraus, wobei der ballonförmige Bereich durch die Reichweite der Elektronenstrahlung und die Mehrfachstreuung der Elektronen definiert wird. Die Lichtstrahlung setzt sich isotrop in den Raum fort. Zur Erfüllung der Aufgabe der vorliegenden Erfindung ist es ausreichend, nur ein sehr kleines Flächenelement des ballonförmigen Bereichs zu überwachen. Beispielsweise ist die Überwachung eines Flächenelementes einer Größe von ca. $0,2\,cm^2$, entsprechend einem kreisrunden Beobachtungsfenster mit einem Durchmesser von 5 mm ausreichend.

Geht man von einem Detektor mit einer sensitiven Fläche von $0,2\,cm^2$ aus (5 mm Durchmesser), der in einem Abstand von 30 cm vom Zentrum des Rekombinationsbereiches sitzt und auf das Zentrum blickt, dann sieht der Detektor aufgrund des Verhältnisses beider Raumwinkel, nämlich des vollen Raumwinkels von $4\pi r^2$ in welchem die Lichtstrahlung emittiert wird und wobei r dem Abstand des Zentrums des Rekombinationsbereiches zum Ort des Detektors entspricht, und des Raumwinkels, den die Detektorfläche in Bezug auf das Zentrum des Strahlungsemissionsvolumens einnimmt, lediglich den 57600 Teil der $1,5 \cdot 10^{17}$ Photonen die pro Sekunde als Lichtstrahlung emittiert werden.

Lichtempfindliche Dioden gibt es für einen sehr breiten Empfindlichkeitsbereich, der bereits bei einer Wellenlänge von etwa 200 nm anfängt und der bis über den sichtbaren Bereich hinaus geht. Die Ansprechwahrscheinlichkeit solcher strahlungsempfindlicher Bausteine oder Bauelemente (lichtempfindliche Dioden, strahlungsempfindliche Dioden, CCD-Chips, CMOS-Chips, Photodioden, Phototransistoren, Photowiderstände) liegt bei typisch deutlich höher als 50 %, d. h. mindestens jedes 2. Quant wird auch nachgewiesen, wodurch der Detektor im vorliegenden Beispiel $1,3 \cdot 10^{12}$ Lichtquanten pro Sekunde nachweist.

Eine sinnvolle Kontrolle des Elektronenstroms erfordert ein Kontrollsignal mit einer Genauigkeit von etwa 0,001 bei einer Abtastrate von $10^4$ pro Sekunde. Das bedeutet, man benötigt einen Elektronenstrommonitor der alle 100 ps einen Ist-Wert mit einem Soll-Wert vergleicht, wobei der Ist-Wert eine Genauigkeit von 1 ‰ haben sollte.

Da die Lichtquanten in statistischer Weise und in hoher Zahl emittiert werden, nämlich $1,3 \cdot 10^8$ Lichtquanten pro 100 pts, die im Detektor dann in einen Strom umgewandelt werden, lässt sich auf einfache Weise der Fehler AI des Stromsignals I berechnen.

Aus der Mathematik ist allgemein bekannt, dass die Genauigkeit AI statistisch auftretender Ereignisse - im vorliegenden Fall also die Genauigkeit des gemessenen bzw. ermittelten Wertes für die Stärke des Elektronenstrahls gleich der "Wurzel der Ereignisse" bezogen auf die "Gesamtzahl der Ereignisse" ist. Es gilt als

$$\Delta I = \frac{\sqrt{n}}{n}$$

mit n = Anzahl der Ereignisse

Daher wird bei einer solch hohen Anzahl von Ereignissen (Anzahl der Ereignisse = $1,3 \cdot 10^8$) ein relativer Fehler des Stromsignals von $8,8 \cdot 10^{-8}$ für Abtastzeiten 100 $\mu$s erreicht. Dieser Wert gilt als exzellent und wird wahrscheinlich von keinem anderen Nachweisverfahren erreicht.

Die beschriebene Abschätzung erfordert, dass der Überwachungsbereich frei von jeglichem Tageslicht ist, damit das zu überwachende Signal nicht auf einem hohen Untergrundsignal sitzt. Dazu muss der zu überwachende Bereich so ausgestaltet sein, dass dort kein Fremdlicht, also beispielsweise Tageslicht oder Licht aus Beleuchtungseinrichtungen, störend wirken könnte.

Eine weitere Ausgestaltung des Detektors nutzt eine Optik aus, mit welcher der erfassbare Raumwinkelbereich eingeschränkt wird und welche auch evtl. vorhandene Streustrahlung des Tageslichtes abblendet.

Dadurch kann zwar - bedingt durch die Einschränkung des Blickfeldes des Detektors - die Anzahl der einfallenden Lichtquanten auf die empfindliche Diode reduziert werden, jedoch würde selbst bei einem auf den Detektor einfallenden Photonenfluss von lediglich $1,3 \cdot 10^8$ Quanten je Zeitintervall von 100 $\mu$s die erreichte statistische Genauigkeit immer noch bei $8,8 \cdot 10^{-4}$ liegen.

In einer weiteren technischen Ausgestaltung des Detektors wird ein Farbfilter vor den Detektor geschaltet, der einen kleinen Spektralbereich zum Detektor passieren lässt. Damit kann der Spektralbereich derart eingeschränkt werden, dass man nur charakteristische Emissionslinien des Plasmas zur Stromwandlung in den Detektor passieren lasst. Diese Ausführungsform ist besonders interessant, wenn der Bereich, in dem der Elektronenstrahl den Packstoff behandelt, mit einem reinen Gas geflutet wird. Beispielsweise wird der Behandlungsbereich mit Stickstoff oder Argon geflutet, um Packstoffmodifikationen durch den Sauerstoffanteil der Luft zu vermeiden.

Für eine weitere Ausführungsform dieses Ausführungsbeispiels ist vorgesehen, das Farbfilter derart auszulegen, dass man nur auf Emissionslinien des Stickstoffs oder des Argons sensitiv ist, um die Elektronenstrahlintensität zu überwachen.

Für eine weitere Ausführungsform dieses Ausführungsbeispiels ist vorgesehen, das Farbfilter derart auszulegen, dass man ausschließlich auf die Emissionslinien des Sauerstoffs sensitiv ist, um ein Eindringen von Sauerstoff in den Behandlungsbereich zu überwachen.

Weiterhin kann der Detektor mit einer elektronischen Schaltung, beispielsweise einer Schaltung zur Überwachung des Elektronenstrahls, bzw. des Elektronenstroms kombiniert werden, wobei vorzugsweise die Stärke des Elektronenstroms erfasst werden kann.

Dabei ist es von besonderem Vorteil, wenn es die elektronische Schaltung gestattet, einen oder mehrere, vorzugsweise beliebig wählbare Grenzwerte oder Schaltpunkte vorgeben zu können, wobei dann, wenn die Ist-Stärke des Elektronenstroms diese Schaltpunkte erreicht, unter- oder überschreitet bestimmte Aktionen ausgelöst werden.

Beispielsweise kann ein erster Schaltpunkt verbunden mit dem Signal "Elektronenstrom eingebrochen" vorgesehen sein. Dabei kann das Signal "Elektronenstrom eingebrochen" auch lediglich erst dann ausgegeben werden, wenn der Elektronenstrom für eine gewisse Zeit unterschritten wird.

Außerdem kann ein zweiter Schaltpunkt vorgesehen sein, wobei dieser zweite Schaltpunkt höher liegt als der erste Schaltpunkt. Der zweite Schaltpunkt wird derart mit dem ersten Schaltpunkt verglichen, dass das Signal "Elektronenstrom eingebrochen" nur ausgegeben wird, wenn der zweite Schaltpunkt nach einer gewissen, einstellbaren Zeit nicht wieder überschritten wird.

Ein Detektor zur messtechnischen Erfassung dieser Strahlung kann beispielsweise mit einer Fläche von etwa 0,2 cm$^2$ ausgebildet werden. Dies entspricht bei einer kreisartigen Detektorfläche einem Durchmesser von etwa 5 mm. Ein typischer Abstand zum Zentrum des Rekombinationsbereiches und somit zur Mittellinie des Elektronenstrahles beträgt etwa 30 cm. Allerdings können auch Sensoren mit anderen Querschnitten eingesetzt werden und andere Abstände zum Rekombinationsbereich gewählt werden.

Eine typische Ausbildung eines derartigen Detektors erfolgt in Form von lichtempfindlichen Halbleiterdioden. Derartige Dioden weisen einen Empfindlichkeitsbereich auf, der sich über einen Wellenlängenbereich von etwa 200 nm bis zum Bereich des sichtbaren Lichtes hin erstreckt. Ebenfalls ist es möglich, als Detektoren CCD-Chips einzusetzen.

Gemäß einer bevorzugten konstruktiven Realisierung wird der Detektor derart angeordnet, dass zumindest im Wesentlichen ausschließlich die vom Elektronenstrahl emittierte elektromagnetische Strahlung erfasst wird. Der Sensor wird hierzu derart abgeschattet, dass Tageslicht oder Licht aus Beleuchtungseinrichtungen nur in einem höchstens unwesentlichen Umfang in den Bereich des Detektors gelangen kann.

Gemäß der vorliegenden Erfindung ist der Detektor auch so angeordnet, dass er die durch die Wandung eines transparenten oder durchscheinenden Packstoffes auf das Plasma schaut. Beispielsweise kann der Elektronenstrahl zur Stabilisierung oder Desinfizierung der inneren Packstoffoberfläche einer PET-Flasche oder eines anderen hohlkörperförmigen Packstoffes herangezogen werden und im Hohlkörper des Packstoffes das dazu notwendige Desinfektions- oder Sterilisationsplasma erzeugen. Der Detektor ist dabei so angeordnet, dass er durch die Wandung auf das im Hohlkörper erzeugte Plasma gerichtet ist und damit die Strahlungsemission des im Hohlkörper vorhandenen Plasmas überwacht. Auch bei dieser Ausführungsform kann der Detektor mit einer Optik und/oder einem Farbfilter ausgestattet sein.

Dabei kann der Packstoff beispielsweise kunststoff- oder glasförmig sein, oder auch aus einem anderen Material bestehen, wobei es erforderlich ist, dass der Packstoff für die bei der Rekombination der Elektronen-Ionen-Paare entstehende Strahlung - zumindest aber für Teile dieser Strahlung durchsichtig, durchgängig oder durchscheinend ist.

Bezugszeichenliste:

1.  Elektronenemitter

2.  Austrittsfenster

3.  Grenze des ballonförmigen Bereichs der UV- oder Lichtstrahlung

4.  Licht- oder UV-Quant

5.  ballonförmiger Bereich der UV- oder Lichtstrahlung

6. Filter

7. Optik

8. lichtempfindliches Element

**Patentansprüche**

1. Verfahren zur Überwachung der Intensität eines, während seiner Ausbreitung ein Plasma erzeugenden Elektronenstrahls, wobei zur Erkennung von Änderungen der Intensität des Elektronenstrahles eine indirekt vom Elektronenstrahl erzeugte elektromagnetische Strahlung detektiert und ausgewertet wird und wobei ein zur messtechnischen Erfassung einer indirekt vom Elektronenstrahl erzeugten elektromagnetischen Strahlung ausgebildeter Detektor vorgesehen ist,
**dadurch gekennzeichnet, dass**
der Detektor durch die Wandung eines transparenten oder durchscheinenden Packstoffes auf das Plasma schaut, wobei der Detektor die Strahlung des vom Elektronenstrahl generierten Plasmas detektiert.

2. Verfahren nach Anspruch 1, **dadurch gekennzeichnet, dass** eine elektromagnetische Ultraviolett-Strahlung oder eine elektromagnetische Lichtstrahlung ausgewertet wird.

3. Verfahren nach Anspruch 1 oder 2, **dadurch gekennzeichnet, dass** die Strahlung während einer Ausbreitung des Elektronenstrahles innerhalb von atmosphärischer Luft oder Stickstoff oder Argon ausgewertet wird.

4. Verfahren nach einem der Ansprüche 1 bis 3, **dadurch gekennzeichnet, dass** der Elektronenstrahl zur Keimreduktion im Bereich einer Oberfläche eines Packstoffes verwendet wird.

5. Verfahren nach Anspruch 4, **dadurch gekennzeichnet, dass** die Keimanzahl im Bereich der Oberfläche eines Behälters reduziert wird.

6. Verfahren nach einem der Ansprüche 1 bis 5, **dadurch gekennzeichnet, dass** die vom Elektronenstrahl erzeugte Strahlung von einem Halbleitersensor detektiert wird.

7. Verfahren nach Anspruch 6, **dadurch gekennzeichnet, dass** als Halbleitersensor eine strahlungs- oder lichtempfindliche Diode verwendet wird.

8. Verfahren nach Anspruch 6, **dadurch gekennzeichnet, dass** als Halbleitersensor ein CCD-Chip oder ein CMOS-Chip oder eine Photodiode oder ein Phototransistor oder ein Photowiderstand verwendet wird.

9. Verfahren nach einem der Ansprüche 6 bis 8, **dadurch gekennzeichnet, dass** die elektromagnetische Strahlung, bevor diese zu dem Halbleitersensor gelangt, einen Spektralfilter oder einen Farbfilter passiert hat.

10. Verfahren nach einem der Ansprüche 6 bis 9, **dadurch gekennzeichnet, dass** die von dem Halbleitersensor empfangene Strahlung im Inneren eines Hohlkörpers entsteht.

11. Vorrichtung zur Überwachung der Intensität eines Elektronenstrahles, wobei der Elektronenstrahl während seiner Ausbreitung ein Plasma erzeugt, wobei ein zur messtechnischen Erfassung einer indirekt vom Elektronenstrahl erzeugten elektromagnetischen Strahlung ausgebildeter Detektor vorgesehen ist, und wobei der Detektor mit einer Auswertungseinrichtung zur Erkennung von Änderungen der Intensität der vom Elektronenstrahl erzeugten elektromagnetischen Strahlung verbunden ist,
**dadurch gekennzeichnet, dass**
der Detektor so angeordnet ist, dass er durch die Wandung eines transparenten oder durchscheinenden Packstoffes auf das Plasma schaut, wobei der Detektor die Strahlung des vom Elektronenstrahl generierten Plasmas detektiert.

12. Vorrichtung nach Anspruch 11, **dadurch gekennzeichnet, dass** der Detektor zur Erfassung von elektromagnetischer Ultraviolett-Strahlung oder elektromagnetischer Lichtstrahlung ausgebildet ist.

13. Vorrichtung nach Anspruch 11 oder 12, **dadurch gekennzeichnet, dass** der Detektor im Bereich einer durch Umgebungsluft verlaufenden Ausbreitungsstrecke des Elektronenstrahles angeordnet ist.

14. Vorrichtung nach Anspruch 11 oder 12, **dadurch gekennzeichnet, dass** der Detektor im Bereich einer durch Stickstoff oder Argon verlaufenden Ausbreitungsstrecke des Elektronenstrahles angeordnet ist.

15. Vorrichtung nach einem der Ansprüche 11 bis 14, **dadurch gekennzeichnet, dass** der Detektor als Teil einer Einrichtung zur Keimreduktion im Bereich einer Oberfläche eines Packstoffes ausgebildet ist.

16. Vorrichtung nach einem der Ansprüche 11 bis 14, **dadurch gekennzeichnet, dass** der Detektor als Teil einer Einrichtung zur Keimreduktion im Bereich einer Oberfläche eines Behälters ausgebildet ist.

17. Vorrichtung nach einem der Ansprüche 11 bis 16, **dadurch gekennzeichnet, dass** der Detektor als ein Halbleitersensor ausgebildet ist.

18. Vorrichtung nach Anspruch 17, **dadurch gekennzeichnet, dass** der Halbleitersensor als eine strahlungsempfindliche Diode oder als lichtempfindliche Diode ausgebildet ist.

19. Vorrichtung nach Anspruch 17, **dadurch gekennzeichnet, dass** der Halbleitersensor als CCD-Chip oder als CMOS-Chip oder als Photodiode oder als Phototransistor oder als Photowiderstand ausgebildet ist.

20. Vorrichtung nach einem der Ansprüche 17 bis 19, **dadurch gekennzeichnet, dass** der Halbleitersensor derart angeordnet ist, dass er die im inneren eines Packstoffkörpers entstehende elektromagnetische Strahlung empfangen kann.

**Claims**

1. Method for monitoring the intensity of an electron beam producing a plasma during its propagation, wherein, in order to identify changes in the intensity of the electron beam, an electromagnetic radiation produced indirectly by the electron beam is detected and evaluated, and wherein a detector is provided which is configured for the detection by technical measurement means of electromagnetic radiation which is indirectly produced by the electron beam, **characterised in that**
the detector views the plasma through the wall of a transparent or translucent packing material, wherein the detector detects the radiation generated by the electron beam.

2. Method according to claim 1, **characterised in that** an electromagnetic ultraviolet radiation or an electromagnetic light beam is evaluated.

3. Method according to claim 1 or 2, **characterised in that** the radiation is evaluated during a propagation of the electron beam inside atmospheric air or nitrogen or argon.

4. Method according to any one of claims 1 to 3, **characterised in that** the electron beam is used for the reduction of bacteria in the region of a surface of a packaging material.

5. Method according to claim 4, **characterised in that** the bacteria count in the region of the surface of a container is reduced.

6. Method according to any one of claims 1 to 5, **characterised in that** the radiation produced by the electron beam is detected by a semiconductor sensor.

7. Method according to claim 6, **characterised in that** a radiation-sensitive or light-sensitive diode is used as the semiconductor sensor.

8. Method according to claim 6, **characterised in that**, as the semiconductor sensor, use is made of a CCD chip or a CMOS chip or a photodiode or a phototransistor or a photoresistor.

9. Method according to any one of claims 6 to 8, **characterised in that** the electromagnetic radiation, before reaching the semiconductor sensor, has passed through a spectral filter or a colour filter.

**10.** Method according to any one of claims 6 to 9, **characterised in that** the radiation received by the semiconductor sensor is produced in the interior of a hollow body.

**11.** Device for monitoring the intensity of an electron beam, wherein the electron beam produces a plasma during its propagation, wherein a detector is provided for the detection by technical measuring means of an electromagnetic radiation produced indirectly by the electron beam, and wherein the detector is connected to an evaluation device for identifying changes in the intensity of the electromagnetic radiation produced by the electron beam, **characterised in that** the detector is arranged in such a way that it views the plasma through the wall of a transparent or translucent packing material, wherein the detector detects the radiation generated by the electron beam.

**12.** Device according to claim 11, **characterised in that** the detector is configured so as to detect electromagnetic ultraviolet radiation or electromagnetic light radiation.

**13.** Device according to claim 11 or 12, **characterised in that** the detector is arranged in the region of a propagation path of the electron beam running through ambient air.

**14.** Device according to claim 11 or 12, **characterised in that** the detector is arranged in the region of a propagation path of the electron beam running through nitrogen or argon.

**15.** Device according to any one of claims 11 to 14, **characterised in that** the detector is configured as a part of an apparatus for reducing bacteria in the region of a surface of a container.

**16.** Device according to any one of claims 11 to 14, **characterised in that** the detector is configured as a semiconductor sensor.

**17.** Device according to claim 11 to 16, **characterised in that** the semiconductor sensor is configured as a radiation-sensitive diode or as a light-sensitive diode.

**18.** Device according to claim 17, **characterised in that** the semiconductor sensor is formed as a radiation-sensitive diode or a light-sensitive diode.

**19.** Device according to claim 17, **characterised in that** the semiconductor sensor is formed as a CCD chip or a CMOS chip or as a photodiode or as a phototransistor or as a photoresistor.

**20.** Device according to any one of claims 17 to 19, **characterised in that** the semiconductor sensor is arranged in such a way that it can receive electromagnetic radiation occurring in the interior of a packaging material body.

**Revendications**

**1.** Procédé servant à surveiller l'intensité d'un faisceau électronique produisant au cours de sa propagation un plasma, dans lequel un rayonnement électromagnétique produit indirectement par le faisceau électronique est détecté et analysé aux fins de l'identification de modifications de l'intensité du faisceau électronique et dans lequel un détecteur réalisé afin de relever par une technique de mesure un rayonnement électromagnétique produit indirectement par le faisceau électronique est prévu, **caractérisé en ce que** le détecteur examine le plasma à travers la paroi d'un matériau d'emballage transparent ou translucide, dans lequel le détecteur détecte le rayonnement du plasma généré par le faisceau électronique.

**2.** Procédé selon la revendication 1, **caractérisé en ce qu'**un rayonnement ultraviolet électromagnétique ou un rayonnement lumineux électromagnétique est analysé.

**3.** Procédé selon la revendication 1 ou 2, **caractérisé en ce que** le rayonnement est analysé au cours d'une propagation du faisceau électronique à l'intérieur de l'air atmosphérique ou de l'azote ou de l'argon.

**4.** Procédé selon l'une quelconque des revendications 1 à 3, **caractérisé en ce que** le faisceau électronique est utilisé afin de réduire les germes dans la zone d'une surface d'un matériau d'emballage.

**5.** Procédé selon la revendication 4, **caractérisé en ce que** le nombre de germes est réduit dans la zone de la surface d'un contenant.

**6.** Procédé selon l'une quelconque des revendications 1 à 5, **caractérisé en ce que** le rayonnement produit par le faisceau électronique est détecté par un capteur à semi-conducteur.

**7.** Procédé selon la revendication 6, **caractérisé en ce qu'**est utilisée en tant que capteur à semi-conducteur une diode sensible au rayonnement ou sensible à la lumière.

**8.** Procédé selon la revendication 6, **caractérisé en ce qu'**est utilisé(e) en tant que capteur à semi-conducteur une puce CCD ou une puce CMOS ou une photodiode ou un phototransistor ou une photorésistance.

**9.** Procédé selon l'une quelconque des revendications 6 à 8, **caractérisé en ce que** le rayonnement électromagnétique a traversé un filtre spectral ou un filtre de couleur avant qu'il n'arrive au capteur à semi-conducteur.

**10.** Procédé selon l'une quelconque des revendications 6 à 9, **caractérisé en ce que** le rayonnement reçu par le capteur à semi-conducteur se forme à l'intérieur d'un corps creux.

**11.** Dispositif servant à surveiller l'intensité d'un faisceau électronique, dans lequel le faisceau électronique produit au cours de sa propagation un plasma, dans lequel un détecteur réalisé afin de relever par une technique de mesure un rayonnement électromagnétique produit indirectement par le faisceau électronique est prévu, et dans lequel le détecteur est relié à un système d'analyse servant à identifier des modifications de l'intensité du rayonnement électromagnétique produit par le faisceau électronique,
**caractérisé en ce que**
le détecteur est disposé de telle manière qu'il examine le plasma à travers la paroi d'un matériau d'emballage transparent ou translucide, dans lequel le détecteur détecte le rayonnement du plasma généré par le faisceau électronique.

**12.** Dispositif selon la revendication 11, **caractérisé en ce que** le détecteur est réalisé afin de relever un rayonnement ultraviolet électromagnétique ou un rayonnement lumineux électromagnétique.

**13.** Dispositif selon la revendication 11 ou 12, **caractérisé en ce que** le détecteur est disposé dans la zone d'un parcours de propagation, s'étendant à travers l'air environnant, du faisceau électronique.

**14.** Dispositif selon la revendication 11 ou 12, **caractérisé en ce que** le détecteur est disposé dans la zone d'un parcours de propagation, s'étendant à travers l'azote ou l'argon, du faisceau électronique.

**15.** Dispositif selon l'une quelconque des revendications 11 à 14, **caractérisé en ce que** le détecteur est réalisé en tant que partie d'un système servant à réduire les germes dans la zone d'une surface d'un matériau d'emballage.

**16.** Dispositif selon l'une quelconque des revendications 11 à 14, **caractérisé en ce que** le détecteur est réalisé en tant que partie d'un système servant à réduire les germes dans la zone d'une surface d'un contenant.

**17.** Dispositif selon l'une quelconque des revendications 11 à 16, **caractérisé en ce que** le détecteur est réalisé sous la forme d'un capteur à semi-conducteur.

**18.** Dispositif selon la revendication 17, **caractérisé en ce que** le capteur à semi-conducteur est réalisé sous la forme d'une diode sensible au rayonnement ou sous la forme d'une diode sensible à la lumière.

**19.** Dispositif selon la revendication 17, **caractérisé en ce que** le capteur à semi-conducteur est réalisé sous la forme d'une puce CCD ou sous la forme d'une puce CMOS ou sous la forme d'une photodiode ou sous la forme d'un phototransistor ou sous la forme d'une photorésistance.

**20.** Dispositif selon l'une quelconque des revendications 17 à 19, **caractérisé en ce que** le capteur à semi-conducteur est disposé de telle manière qu'il peut recevoir le rayonnement électromagnétique se formant à l'intérieur d'un corps de matériau d'emballage.

Stand der Technik.

Fig. 7

Fig. 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **H. KUCHLING.** Taschenbuch der Physik. Carl Hanser Verlag, vol. 16, 572